# EUROPEAN PATENT APPLICATION

(11) **EP 2 465 474 A1**
(43) Date of publication of application: **20.06.2012**
(21) Application number: 11189002.6
(22) Date of filing: 14.11.2011
(51) Int. Cl.: A61F 2/04, A61F 2/90

(54) **Urethral stent for the prostate**

(30) Priority: 16.12.2010 KR 20100128990
(71) Applicant: Taewoong Medical Co., Ltd., Kimpo-si Kyonggi do 415-873 (KR); Shin, Kyong-Min, Seoul (KR)
(72) Inventor: Shin, Kyong-Min, Seoul (KR)
(74) Representative: McCartney, Jonathan William

(57) **Abstract**

A urethral stent (13) for the prostate including a first stent section (11) placed into an upper portion of the urethra passing through the prostate, a second stent section (12) placed into a lower portion of the urethra below a urinary sphincter muscle, the first and second stent sections being formed of a shape-memory alloy wire, and at least one connection filament (19) is provided at a middle portion of the urethra passing through the urinary sphincter muscle so as to connect the first and second stent sections, thereby preventing the stent from moving to the upper or lower portions of the urethra.

## Description

### BACKGROUND OF THE INVENTION

### Field of the Invention

The present invention relates, in general, to a urethral stent for the prostate, and more particularly, to a urethral stent for the treatment of urethral stricture in which the urethra is compressed by the enlargement of a prostate, making it difficult for the prostate to urinate.

### Description of the Related Art

Conventionally, a urethral stent 1 or a tube for the enlargement of the contracted urethra has been placed into the urethra in order to enlarge a urethra that has contracted because of prostate hypertrophy.

That is, the urethral stent 1 is first coated with silicon 2 or the like and then is placed into the urethra 2 so as to enlarge the contracted urethra 2 that has contracted because of hypertrophy, allowing the prostate to urinate easily.

However, since the urethral stent 1 is placed into the urethra 2, there is the problem of it sliding up or down to the bladder 6 when a user urinates so that it moves away from the initial position at which it was placed.

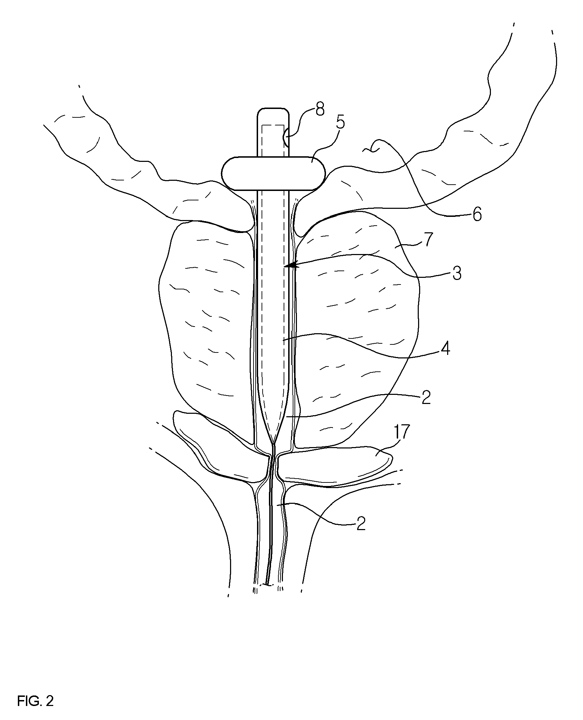

To solve this problem, the tube 3 for the enlargement of the urethra is provided as shown in FIG. 2.

The tube is configured such that an enlarged part 5 is provided in the middle of a hollow tube part 4, that is, between the bladder 6 and the urethra 2. The enlarged part 5 prevents it from moving away toward the urethra 2.

However, since the enlarged part 5 of the tube 3 is placed in the bladder 6, a user feels aggravation in his bladder 6. In addition, since the tube 3 is configured such that a urine outlet 8 which discharges the urine through the hollow tube part 4 is provided in the upper side of the hollow tube part 4, some urine remains behind, or otherwise the user may also always feel as if there is remaining urine. Further, the tube has a very narrow cavity that is ready to be blocked.

### SUMMARY OF THE INVENTION

Accordingly, the present invention has been made keeping in mind the above problems occurring in the related art, and an object of the present invention is to propose a urethral stent for the prostate, which is placed into the urethra such that it cannot be dislocated from the initial position at which it was placed, and does not slip along the urethra even when a user urinates.

In order to achieve the above object, according to one aspect of the present invention, there is provided a urethral stent for the prostate including: a first stent section placed into an upper portion of the urethra passing through the prostate; a second stent section placed into a lower portion of the urethra below a urinary sphincter muscle, the first and second stent sections being formed of a shape-memory alloy wire; and at least one connection filament provided at a middle portion of the urethra passing through the urinary sphincter muscle so as to connect the first and second stent sections, thereby preventing the stent from moving to the upper and lower portions of the urethra.

According to the present invention, the urethral stent is placed into the urethra, thereby having the effect of enlarging the contracted urethra that was contracted by prostate hypertrophy, allowing a user to easily urinate and also preventing the stent from becoming dislocated from the initial position at which it was placed, even by an external force such as urine.

### BRIEF DESCRIPTION OF THE DRAWINGS

The above and other objects, features and advantages of the present invention will be more clearly understood from the following detailed description when taken in conjunction with the accompanying drawings, in which:

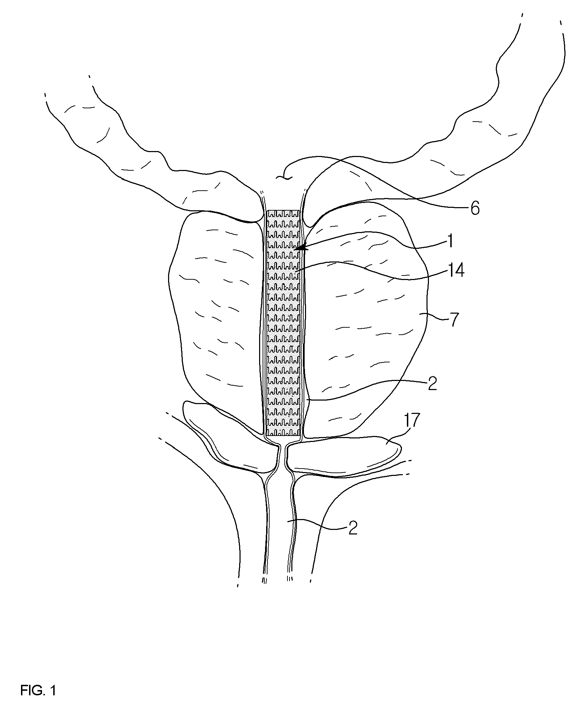

FIGS. 1 and 2 are views showing the respective states of a conventional stent and a urethral enlargement tube being implanted to the prostate;
FIG. 3 is a view showing a urethral stent for the prostate according to an embodiment of the present invention;
FIG. 4 is a view showing a urethral stent for the prostate according to another embodiment of the present invention; and
FIG. 5 is a view showing the state of the urethral stent being inserted into the urethra and the operation of the urethral stent.

### DETAILED DESCRIPTION OF THE INVENTION

Reference will now be made in greater detail to a preferred embodiment of the invention, an example of which is illustrated in the accompanying drawings. Wherever possible, the same reference numerals will be used throughout the drawings and the description to refer to the same or like parts.

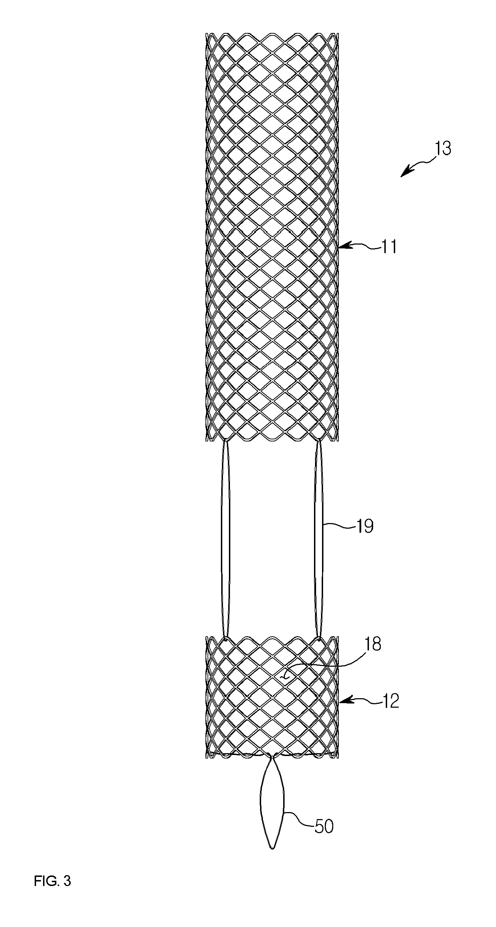

In FIG. 3, a urethral stent 13 for the treatment of urethral stricture caused by an enlarged prostate includes first and second stent sections 11 and 12 which are formed into a net type barrel with a shape-memory alloy wire, and one or more connection filaments 19 provided between the first and second stent sections at regular intervals so as to connect the first and second stent sections.

Unexplained reference numeral 50 is a connection hook that is used to facilitate the removal of the urethral stent 13 which was implanted to the prostate.

Here, the connection filament 19 connecting the first and second stent sections 11 and 12 may use not only a conventional one, but also a metal wire which is not harmful to a human body and is mechanically and chemically resistant to urine.

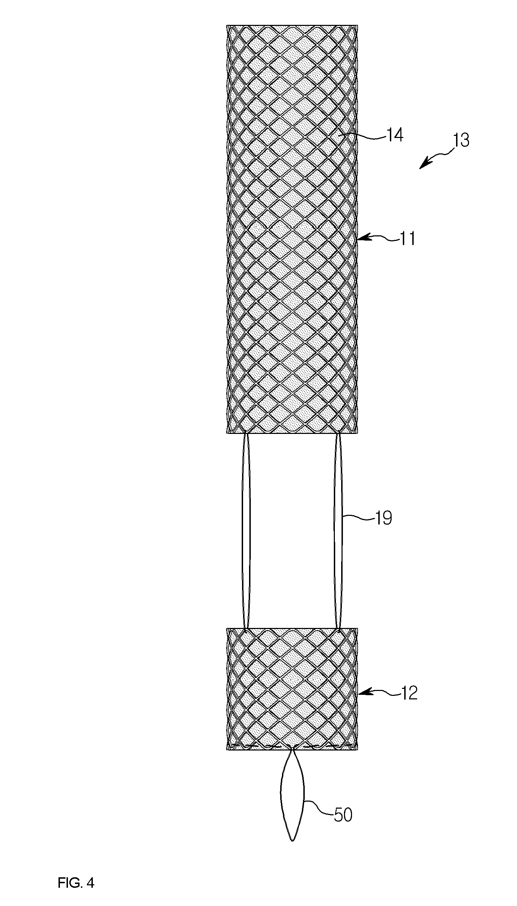

Further, as shown in FIG. 4, inner or outer surfaces of the first and second stent sections 11 and 12 formed of a net of shape-memory alloy wire are coated with a biocompatible cladding 14 such as silicon or PTFE.

The cladding 14 of the first stent section 11 is provided in order to prevent a portion of the prostate 7 from penetrating the first stent section 11 through the mesh holes 18. This is because the urethral stent 13 is implanted into the urethra 2 at a position where the enlarged prostate 7 exists, and the urethral stent pushes against the enlarged prostate 7 at its original position so as to enlarge the urethra.

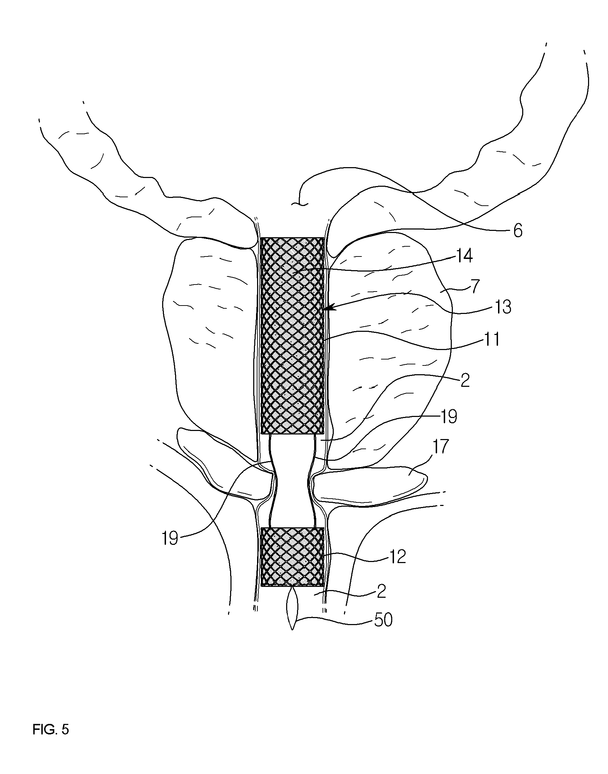

The urethral stent 13 is generally implanted using a catheter in such a manner as is shown in FIG. 5: the first stent section 11 is placed into an upper portion of the urethra 2 where the enlarged prostate 7 exists, so as to push against the enlarged prostate 7 and secure the urethra 2, the second stent section 12 is placed into a lower portion of the urethra below a urinary sphincter muscle 17, and the connection filaments 19 connecting the first and second stent sections are placed into a middle portion of the urethra where the urinary sphincter muscle 17 exists.

In the meantime, the urinary sphincter muscle 17 is located around an outer circumference of the middle portion of the urethra 2 and serves to open or close the urethra 2 so as to regulate urination, so that the width of the middle portion of the urethra 2 near the urinary sphincter muscle is narrower than the other portion of the urethra 2.

Thus, the first stent section 11 and the second stent section 12 are respectively placed into the urethra 2 above and below the urinary sphincter muscle 17, so that the first stent section 11 cannot move downwards, passing through the narrow urethra 2 near the urinary sphincter muscle 17, and the second stent section 12 cannot also move upwards, passing through the narrow urethra 2 near the urinary sphincter muscle 17. Further, the connection filaments 19 are placed near the narrow urethra 2 while connecting the first and second stent sections 11 and 12, so that the first and second stent sections 11 and 12 can be prevented from becoming displaced from their initial positions where they were first implanted.

As described before, the urethral stent 13 is placed into the urethra, thereby having the effect of enlarging the contracted urethra that was contracted by prostate hypertrophy, allowing a user to easily urinate and also preventing the stent from becoming displaced from the initial position where it was placed, even by an external force or urine.

That is, the urethral stent according to the present invention can be implanted to the prostate because it is configured such that two stent sections of the above construction are disposed a distance apart from each other and are integrally connected by connection filaments or other wires having similar functions.

Although a preferred embodiment of the present invention has been described for illustrative purposes, those skilled in the art will appreciate that various modifications, additions and substitutions are possible, without departing from the scope and spirit of the invention as disclosed in the accompanying claims.

## Claims

1. A urethral stent for the prostate comprising: first and second stent sections which are spaced a distance apart and are formed into a net type barrel with a shape-memory alloy wire; and one or more connection filaments provided between the first and second stent sections so as to connect the first and second stent sections.

2. The urethral stent for the prostate according to claim 1, wherein the first stent section is placed into an upper portion of the urethra above a urinary sphincter muscle, the second stent section is placed into a lower portion of the urethra below the urinary sphincter muscle, and the connection filament is provided into a middle portion of the urethra near the urinary sphincter muscle.

3. The urethral stent for the prostate according to claim 1, wherein inner or outer surfaces of the first and second stent sections are coated with a biocompatible cladding formed of silicon or PTFE.

4. The urethral stent for the prostate according to any one of claims 1 to 3, wherein the connection filament connecting the first and second stent sections is a biocompatible metal wire which is mechanically and chemically resistant to urine.
